# EUROPEAN PATENT SPECIFICATION

(11) **EP 2 485 632 B1**
(45) Date of publication and mention of the grant of the patent: **20.04.2016**
(21) Application number: 10771594.8
(22) Date of filing: 07.10.2010
(51) Int. Cl.: A61B 1/00, A61B 17/00

(54) **LAPAROSCOPIC INSTRUMENT WITH ATTACHABLE END EFFECTOR**
LAPAROSKOPISCHES INSTRUMENT MIT BEFESTIGBAREM GREIFORGAN
INSTRUMENT LAPAROSCOPIQUE DOTÉ D'UN EFFECTEUR TERMINAL QUI PEUT S'ATTACHER

(30) Priority: 09.10.2009 US 576565; 09.10.2009 US 576546; 09.10.2009 US 576578
(43) Date of publication of application: 15.08.2012
(73) Proprietor: Ethicon Endo-Surgery, Inc., Cincinnati, OH 45242 (US)
(72) Inventor: NOBIS, Rudolph, H., Mason, OH 45040 (US); SPIVEY, James, T., Cincinnati, OH 45226 (US); HUEY, Kevin, M., Cincinnati, OH 45202 (US); HESS, Christopher, J., Cincinnati, OH 45206 (US); CONLON, Sean, P., Loveland, OH 45140 (US)
(74) Representative: Tunstall, Christopher Stephen
(86) International application number: PCT/US2010/051812
(87) International publication number: WO 2011/044353

(56) References cited:
- EP-A1- 1 709 900
- JP-A- 2005 261 734
- US-A- 6 059 719
- US-A1- 2008 243 106

## Description

### BACKGROUND

The present invention relates in general to surgical devices, and more particularly to minimally invasive surgery.

Surgical procedures are often used to treat and cure a wide range of diseases, conditions, and injuries. Surgery often requires access to internal tissue through open surgical procedures or endoscopic surgical procedures. The term "endoscopic" refers to all types of minimally invasive surgical procedures including laparoscopic, arthroscopic, natural orifice intraluminal, and natural orifice transluminal procedures. Endoscopic surgery has numerous advantages compared to traditional open surgical procedures, including reduced trauma, faster recovery, reduced risk of infection, and reduced scarring. Endoscopic surgery is often performed with an insufflatory fluid present within the body cavity, such as carbon dioxide or saline, to provide adequate space to perform the intended surgical procedures. The insufflated cavity is generally under pressure and is sometimes referred to as being in a state of pneumoperitoneum. Surgical access devices are often used to facilitate surgical manipulation of internal tissue while maintaining pneumoperitoneum. For example, trocars are often used to provide a port through which endoscopic surgical instruments are passed. Trocars generally have an instrument seal, which prevents the insufflatory fluid from escaping while an instrument is positioned in the trocar. Document US2008/243106 discloses a surgical device that allows a surgeon to remotely and selectively attach various interchangeable surgical end effectors to a shaft located within a patient's body, thus allowing the surgeon to perform various procedures without the need to remove the shaft from the patient's body. The device can be configured to allow multiple end effector to be remotely attached and detached from the distal end of the shaft. For example, a surgeon can actuate an actuation mechanism on the proximal end of the shaft to mate one of the end effectors to the distal end of the shaft without assistance from other tools and devices.

While surgical access devices are known, no one has previously made or used the surgical devices in accordance with the present invention.

### BRIEF DESCRIPTION OF DRAWINGS

While the specification concludes with claims which particularly point out and distinctly claim the invention, it is believed the invention will be better understood from the following description taken in conjunction with the accompanying drawings illustrating some non-limiting examples of the invention. Unless otherwise indicated, the figures are not necessarily drawn to scale, but rather to illustrate the principles of the invention.
Fig. 1 depicts a surgical procedure with an instrument and loader holding an end effector;

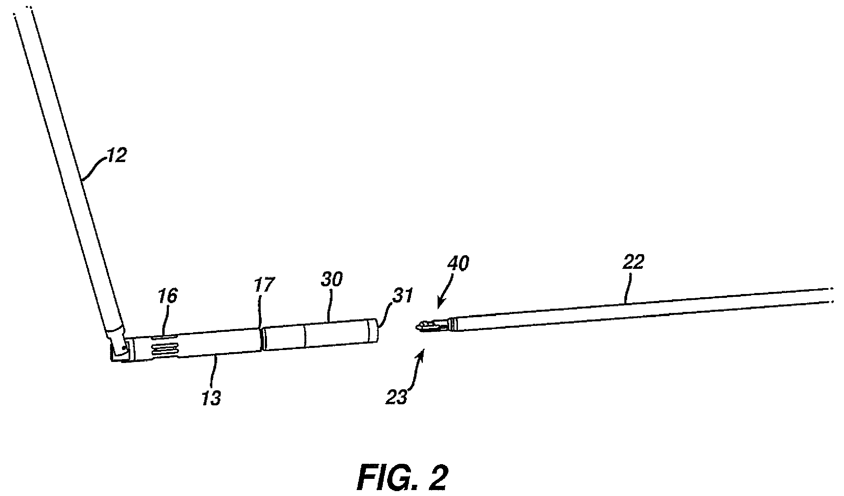

Fig. 2 depicts a close-up view of the distal ends of the instrument and loader of Fig. 1;

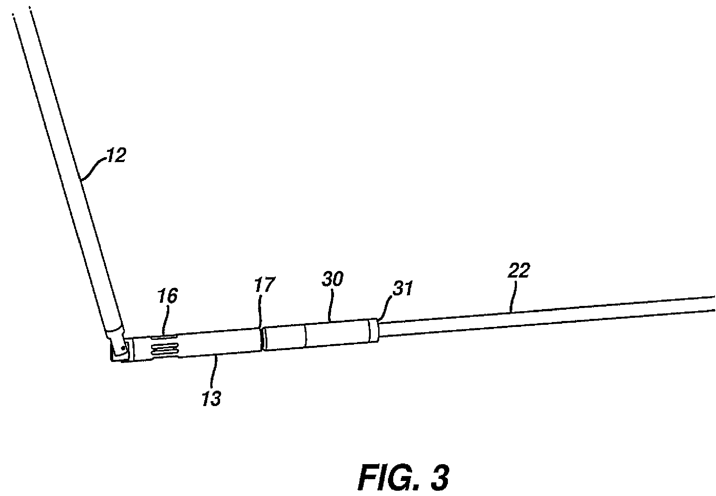

Fig. 3 depicts an instrument being inserted into an end effector;
Fig. 4 depicts an instrument attached to an end effector and being withdrawn from a loader;
Fig. 4A depicts a loader with removable distal end;
Fig. 5 depicts an isometric close-up view of the distal end of an instrument in a locked position;
Fig. 6 depicts an isometric close-up view of the distal end of an instrument in an unlocked position;
Fig. 7 depicts an isometric cross-sectional view of the distal end of an instrument attached to an end effector;
Fig. 8 depicts an isometric cross-sectional view of the distal end of an instrument attached to an end effector in a pushed-off configuration;
Fig. 9 depicts an instrument handle;
Fig. 10 depicts a bi-polar jawed end effector;
Fig. 11 depicts a cutting shears end effector;
Fig. 12 depicts a Maryland dissector end effector; and
Fig. 13 depicts an ultrasonic shears end effector;

### DETAILED DESCRIPTION

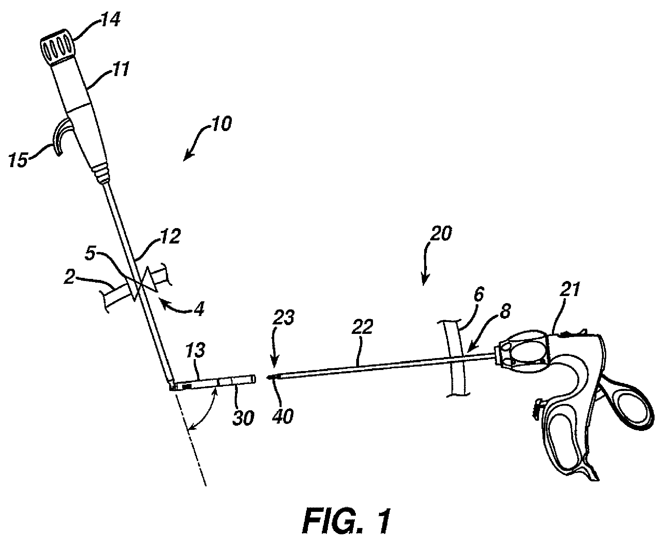

As shown in Fig. 1, instrument (20) comprises an elongate shaft (22) passing through an incision (8) of a tissue wall (6). A loader (10) comprises an elongate shaft (12) passing through an incision (4) of a tissue wall (2). The surgical end effector (30) is selectively attachable in vivo and detachable in vivo to the attachment mechanism (40) located at the distal end (23) of the instrument (20). In this example, the end effector is a jawed tissue grasper, but a variety of other end effectors could be also be used. The end effector (30) may be loaded ex vivo into the distal end (13) of the shaft (12), and then introduced into the surgical field through the incision (4). The loader (10) holds the end effector (30) during the in vivo attachment to and in vivo detachment from the instrument (20). The loader (10) and instrument (20) each includes ex vivo handles (11, 21) attached to the proximal ends of the shafts (12, 22) that enable surgeons to use the devices.

The tissue wall (2, 6) anatomies will vary based on the surgical procedure, but some non-limiting examples include percutaneous incisions into the abdomen, thorax, or pelvis. The incisions (4, 8) may be created with a cutting or puncturing instrument, and will typically be spaced from one another. The tissue walls (2, 6) may be the same or different anatomies. For instance, tissue walls (2, 6) may both be the abdominal wall. In another example, tissue wall (2) could be an organ (e.g., stomach, colon, esophagus, etc.) accessed through a natural orifice, while the incision (8) in tissue wall (6) could be percutaneous. In yet another example, incision (4) may provide access to the abdomen, while the incision (8) may provide access to the pelvis. If pneumoperitoneum is desired, the incisions may include instrument seals, such as those commonly found in trocars. In this example, the instrument seal (5) is schematically shown in incision (4) with the loader (10) passing through the seal (5), while the shaft (22) seals directly with the tissue wall (6) by virtue of the resilience of the tissue without the aid of a sealing device.

The loader shaft (12) in this embodiment is rigid and straight, but the shaft (12) could be curved or flexible, which would be beneficial for natural orifice transluminal introduction of the distal end (13) to the surgical field. The loader (10) may include an articulating distal end (13) controlled by the knob (14). The distal end (13) will typically be introduced and removed through the incision (4) in-line with the shaft (12), and then articulated in vivo to facilitate alignment between the end effector (30) and the shaft (22). The arm (15) is rigidly connected the handle (11) to facilitate grasping of the handle and rotational orientation of the articulated distal end (13) about the shaft (12) axis. In this embodiment, the distal end (13) of the loader (10) comprises a tube opening at the distal tip (17). The tube is dimensioned to receive the end effector (30). The tube (30) includes an engagement feature (16) for holding the end effector (30). While the engagement feature (16) may vary, in this embodiment a plurality of leaf springs provide an interference fit with the end effector (30) to frictionally hold the end effector in the tube. In this embodiment, when the end effector (30) is loaded in the tube, the distal end (32) is positioned in the tube and the proximal end (31) extends from the tube opening (17). This arrangement prevents the jaws of the end effector from opening. After the distal end (23) of the instrument (20) is attached to the proximal end (31) of the end effector (30), the end effector (3) can be pulled from the distal end (13) of the loader (10).

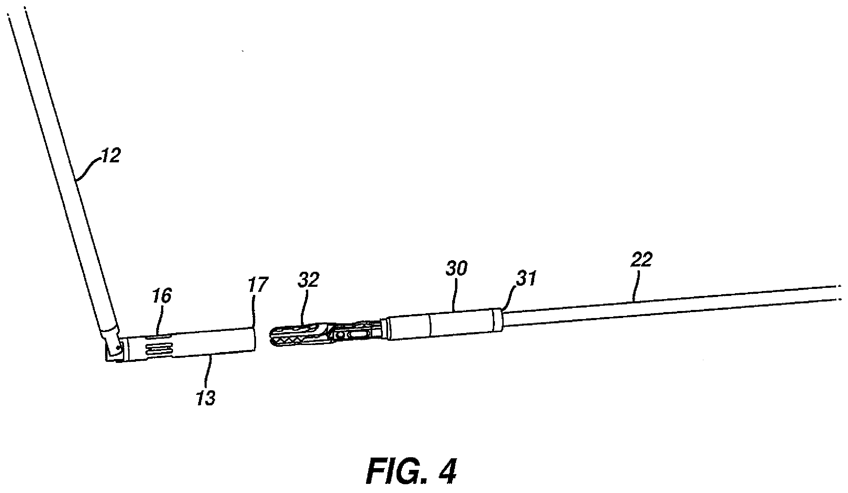

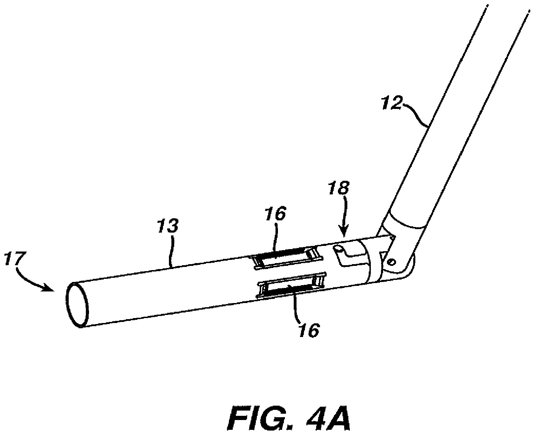

Fig. 4A depicts an alternative embodiment of a loader (10) where the distal end (13) is selectively attachable and detachable to the shaft (12). As shown in this example, this feature is enabled with a bayonet connection (18), but other connections are also contemplated including snap connections, threaded connections, and the like. One advantage of this alternative embodiment is that different distal end (13) configurations may be used to hold end effectors that may not be accommodated by a single sized tube.

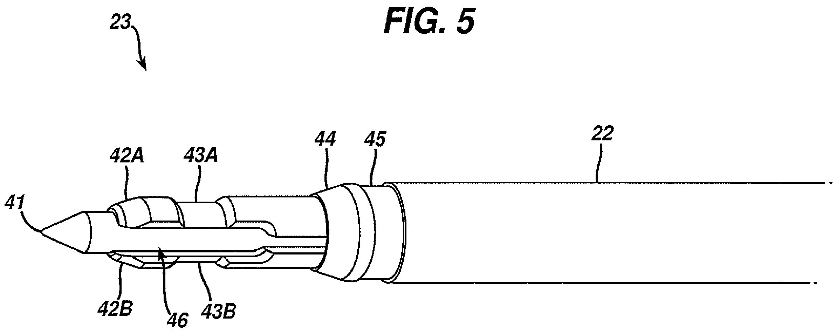

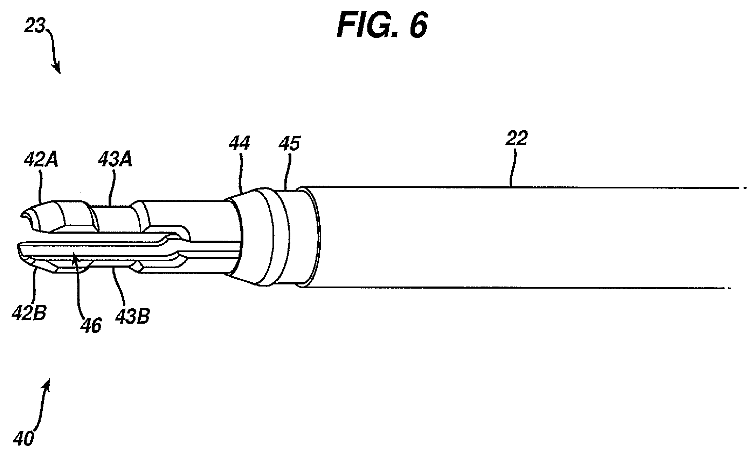

Figs. 5 and 6 depict a detailed view of one embodiment of an attachment mechanism (40) located at the distal end (23) of the shaft (22). The attachment mechanism (40) comprises a mating feature on the shaft (22), which in this embodiment is a circumferential groove (45) positioned on the lateral surface of the shaft (22). The attachment mechanism (40) also comprises arms (42A, 42B) projecting distally from the distal end (44) of the shaft (22). The arms are axially slideable relative the shaft (22) and are resiliently deflectable medially into the gap (46). The arms each comprise a mating feature, which in this embodiment comprises a stepped lateral notch (43A, 43B). An elongate pin (41) is positioned medially relative the arms (42) and is axially slideable relative the arms (42) between a locked position preventing medial deflection of the arms (an example of which is shown in Fig. 5) and an unlocked position allowing medial deflection of the arms (an example of which is shown in Fig. 6). The pin (41) and arms (42) may each slide independently relative the shaft (22).

As shown in the embodiment of Fig. 5, the elongate pin (41) may include a pointed obtruator tip. In this configuration the distal end (23) may be used to puncture through the tissue wall (6). The distal ends of the arms (42) and distal end (44) of the shaft (22) include tapered surfaces to facilitate passing through the incision (8).

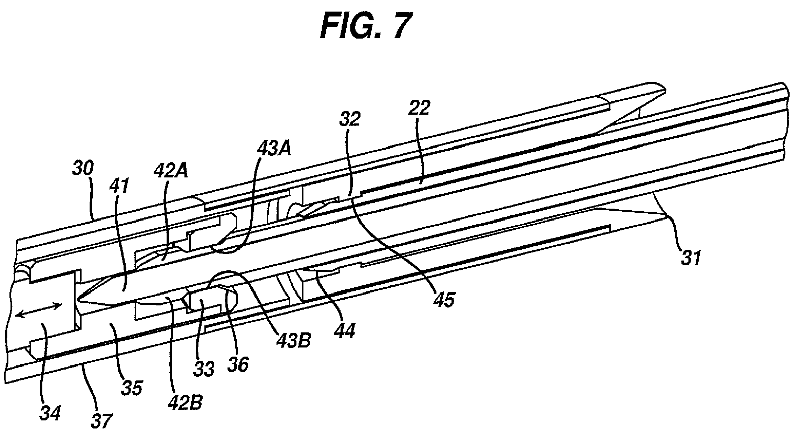

Fig. 7 shows the attachment mechanism (40) attached to the end effector (30). The groove (45) of the shaft (22) mates the rib (32) of the end effector (30) preventing relative axial motion. The lateral grooves (43) of the arms (42) mate the ring (33) of the end effector (30) preventing relative axial motion. The rib (32) is rigidly connected to the outer housing (37) of the end effector (30), and the ring (33) is rigidly connected to the jaw actuator (34) via the coupling (35). Accordingly, axial movement of the arms (42) relative the shaft (22) will cause axial movement of the jaw actuator (34) relative the housing (37), thereby causing the jaws to open and close.

The following describes one method for attaching the end effector (30) to the shaft (22). The distal end (23) is introduced in into the proximal end (31) of the end effector (30) with the pin (41) in the unlocked position. As the arms (42) are advanced axially into the end effector (30), the chamfered lead (36) of the ring (33) medially deflects the arms (42) until the ring (33) is seated into the lateral notches (43). Simultaneously the shaft (22) advances axially into the end effector (30), and the tapered end (44) aligns the rib (32) to seat into the groove (45). In both cases, the surgeon will feel a tactile "snap" indicating proper engagement. Once fully seated in the end effector (30), the pin (41) may be slid to the locked position thereby attaching the end effector (30) to the instrument (20). Once attached, the surgeon may pull the end effector from the loader (10), and the loader (10) may then be removed from the surgical field. The surgeon may then manipulate tissue with the end effector (30) as needed for the surgical procedure.

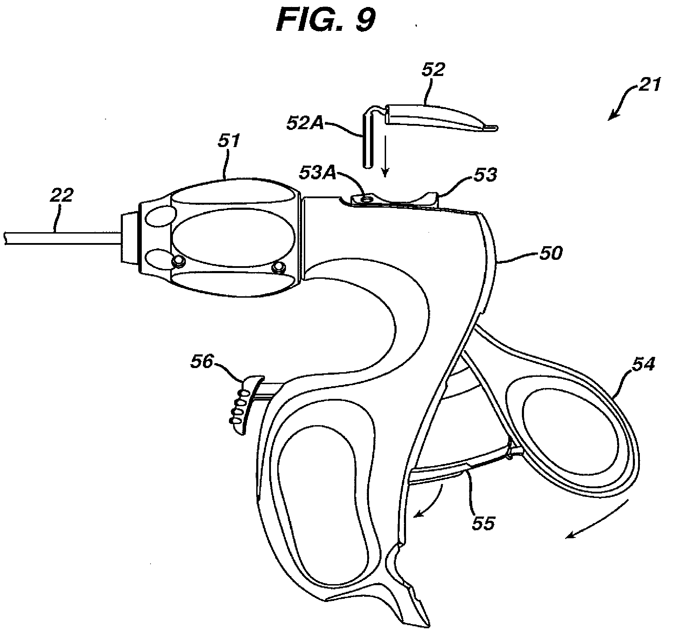

Fig. 9 shows and example of the handle (21) for the instrument (20). The handle (21) includes a base (50). A knob (51) rotates the attachment mechanism (40) about the axis of the shaft (22), which will also rotate an attached end effector (30). The trigger (54) pivots relative the base (50) causing axial movement of the arms (42) and the pin (41) relative the shaft (22). Operation of the trigger (54) will operate the jaws on an attached end effector (30). The latch (55) pivots relative the base (50) between a locked position (as shown in figure) to prevent operation of the trigger (54) and an unlocked position recessed in the base (50). During seating with the end effector (30), the latch (55) may be locked to maintain the same relative axial spacing of the corresponding the mating features (43, 45) as the mating features (33, 32), resulting in resulting in a single "snap" feedback. The trigger lock (56) can lock/unlock the trigger in/from its depressed position. An actuator (53), which in this embodiment is a slider, controls axial movement of the pin (51) relative the arms (42). The distal most position of the actuator (53) relative the base (as shown in the figure) places the pin (51) in its locked position, and the proximal most position places the pin (51) in its unlocked position. The pin lock (52) includes a pin (52A) which went inserted into the hole (53A) maintains the pin (41) and arms (42) in the extended and locked positions as shown in Fig. 5.

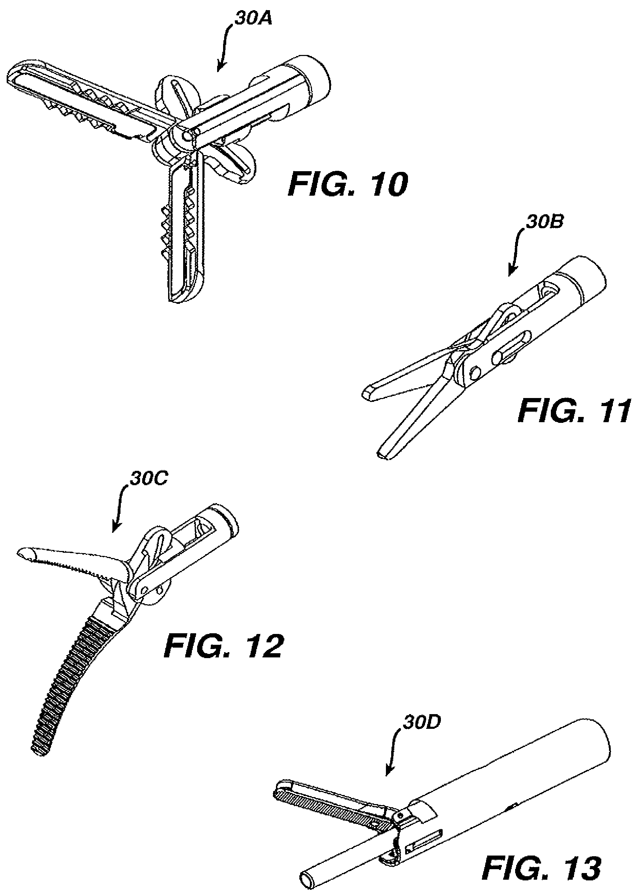

Figs. 10-13 illustrate some non-limiting examples of alternative end effectors (30A-D) that may attached to the distal end (23) of the instrument (20). In addition to the loader (10) and instrument (20), all or a portion of the end effectors (30, 30A, 30B, 30C, 30D) may be bundled as part of a kit so the surgeon may interchange the attached end effector as needed for a surgical procedure. All the end effectors examples shown here have cooperating jaws; however, non-jawed end effectors could also be employed such as hook knives, snares, and the like. In the case of end effectors that require energy, appropriate energy transmission mechanisms known in the art should be added to the handle (21) and shaft (22). For instance, appropriate electrical connections can be added for the bi-polar forceps end effector (30A). Similarly, an ultrasonic transducer and waveguide can be added for the ultrasonic shears end effector (30D).

The following describes one method for using the devices during a laparoscopic surgical procedure. An instrument (20) is obtained and passed through incision (8). The incision (8) may be a precutaneous incision formed at least partially by a puncture formed with the obtruator on the pin (41) in the configuration shown in Fig. 5. The pin lock (52) and latch (55) may be secured to the slider (53) and trigger (54), respectively. After the puncture, the pin lock (52) may be removed.

A loader (10) and end effector (30) are obtained. The end effector (30) may be selected from a plurality of end effectors provided in a kit. The end effector (30) is loading ex vivo into the distal end (13) of the loader (10). The distal end (13) of the loader (10) with the loaded end effector (30) is passed through incision (4). The second incision (4) may also be percutaneous incision spaced from the first incision (8), and may include passing the distal end (13) with the loaded end effector (30) through a trocar. The distal end (13) may be articulated to facilitate orientation between the proximal end (31) of the end effector (30) and the attachment mechanism (40). The actuator (53) is slid proximally to move the pin (41) to its unlocked position. The distal end (23) of the instrument (20) is advanced into the proximal end (31) of the end effector (30) until the respective mating features of the instrument (20) and end effector (30) are engaged. The actuator (53) may then be slid distally thus advancing the pin (41) to its locked position. The end effector (30) has now been attached in vivo to the instrument (20). The end effector (30) may then be pulled from the loader (10) and the latch (55) disengaged from the trigger (54). Tissue is then manipulating by actuating the trigger (54) of the handle (21) to operate the jaws of the end effector (30).

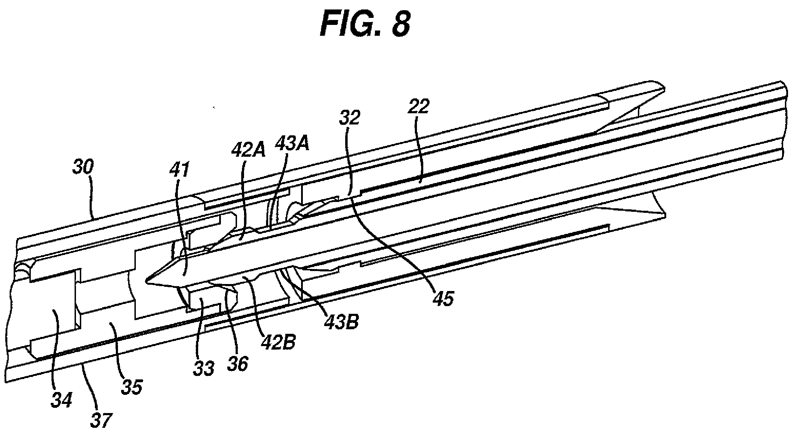

After completing the surgical procedure, the end effector (30) may be detached from the shaft (22). If previously removed, the loader (10) may be reintroduced through the incision (4) into the surgical field. The distal end (32) of the end effector (30) is seated into the distal end (13) of the loader (10), and the pin (41) moved to its unlocked position. The arms (42) are then proximally withdrawn from the ring (33) and the pin (41) is returned to the locked position. Accordingly, the device will be in the configuration depicted in Fig. 8. Distally advancing the arms (42) will push the ring (33) distally till the rib (32) unseats from the groove (45). This unseating may be facilitated by the jaws of the end effector (30) being held in a closed position by the tube in the loader distal end (13). The distal end (23) may then be withdrawn from the end effector (30) thus detaching the end effector (30) from the instrument (20). The end effector will be held in the loader (10) by virtue of the engagement feature (16). Removal of the loader (10) from the surgical field will remove the end effector (30). A different end effector may then be attached to the instrument (20), or the instrument (20) may be withdrawn from the surgical field.

Without limitation, the following describe some of the benefits and advantages of the foregoing devices and methods over the prior art. The end effector (30) may have a much larger diameter than the shaft (22); accordingly, the incision (8) can be smaller compared to more traditional laparoscopic instruments resulting in less pain and scarring, and quicker recovery. This also facilitates a small diameter shaft (22) (even less than 3mm), thus potentially eliminating a trocar in the incision (8). The attachment mechanism (40) provides quick end effector (30) exchanges with the instrument (20), thus decreasing surgical time. The loader (10) also facilitates quick end effector (30) exchanges. A kit of multiple end effectors may reduce instrument costs by consolidating a single shaft (22) and handle (21) for all instruments. Many other benefits will be apparent to those skilled in the art.

Having shown and described various embodiments and examples of the present invention, further adaptations of the devices described herein can be accomplished by appropriate modifications by one of ordinary skill in the art without departing from the scope of the present invention. Several of such potential modifications have been mentioned, and others will be apparent to those skilled in the art. For instance, the specific materials, dimensions, and the scale of drawings will be understood to be non-limiting examples. Accordingly, the scope of the present invention should be considered in terms of the following claims and is understood not to be limited to the details of structure, materials, or acts shown and described in the specification and drawings.

## Claims

1. A surgical device, comprising:
an instrument (20) and a surgical end effector (30) selectively attachable to and detachable from the instrument (20), the instrument (20) comprising:
a) an elongate shaft (22) defining a longitudinal axis, the shaft (22) comprising a distal end, a proximal end and a first mating feature (45) adapted to mate with a first feature (32) on the surgical end effector (30);
b) an arm (42) comprising a second mating feature (43) adapted to mate with a second feature (33) on the surgical end effector, wherein the arm (42) is adapted to be axially slideable relative to the elongate shaft (22) and to be medially deflectable; and
c) an elongate pin (41) positioned medially relative to the arm (42), the elongate pin (41) being axially slideable relative to the arm (42) between a locked position preventing medial deflection of the arm (42) and an unlocked position allowing medial deflection of the arm (42),
wherein the surgical end effector (30) is adapted to be selectively attacheable in vivo to and detacheable in vivo from the second mating feature (43) of the arm (42) by medial deflection of the arm (42).

2. The surgical device of claim 1, wherein
the second mating feature (43) is a lateral notch (43) at the distal end of the elongate shaft (22).

3. The surgical device of claim 2, wherein the second feature (33) on the surgical end effector (30) comprises a ring (33) dimensioned to mate with the lateral notch (43).

4. The surgical device of claim 1, comprising two or more arms (42) circumscribing the elongate pin (41).

5. The surgical device of claim 1, wherein the distal end of the elongate pin (41) comprises an obturator tip.

6. The surgical device of claim 1, further comprising a handle (21) operatively connected to the proximal end of the elongate shaft (22), the handle (21) comprising a trigger (54) controlling the axial movement of the arm (42) and an actuator (53) controlling the axial movement of the elongate pin (41).

7. The surgical device of claim 6, wherein the actuator (53) is lockable.

8. The surgical device of claim 1, wherein the end effector (30) has cooperating jaws that move between open and closed positions in response to axial motion of the arm (42).

9. The surgical device of claim 1, wherein the arm (42) projects distally from the distal end of the elongate shaft (22).

10. A laparoscopic surgical device according to claim 1, comprising:
a plurality of arms (42) that project distally from the distal end of the elongate shaft (22), the arms (42) each comprising a lateral notch (43) as a mating feature, the arms (42) being axially slideable relative the elongate shaft (22) and being medially deflectable;
wherein the elongate pin (41) is positioned medially relative the arms (42), the elongate pin (41) being adapted to be axially slideable relative to the arms (42) between a locked position preventing medial deflection of the arms (42) and an unlocked position allowing medial deflection of the arms (42); and
wherein the surgical end effector (30) is adapted to be selectively attacheable in vivo to and detacheable in vivo from the mating feature (43) of the arms (42), the surgical end effector (30) comprising jaws that open and close in response to the axial movement of the arms (42) when attached to the surgical end effector (30).

11. The laparoscopic surgical device of claim 10, further comprising an obturator tip on
the distal end of the elongate pin (41).

## Patentansprüche

1. Chirurgische Vorrichtung, umfassend:
ein Instrument (20) und ein chirurgisches Greiforgan (30), das selektiv an dem Instrument (20) befestigt und davon entfernt werden kann, wobei das Instrument (20) Folgendes umfasst:
a) einen langgestreckten Schaft (22), der eine Längsachse definiert, wobei der Schaft (22) ein distales Ende, ein proximales Ende und ein erstes Passmerkmal (45) umfasst, das ausgelegt ist, um mit einem ersten Merkmal (32) auf dem chirurgischen Greiforgan (30) zusammenzupassen;
b) einen Arm (42), der ein zweites Passmerkmal (43) umfasst, das ausgelegt ist, um mit einem zweiten Merkmal (33) auf dem chirurgischen Greiforgan zusammenzupassen, wobei der Arm (42) ausgelegt ist, um bezüglich des langgestreckten Schafts (22) axial verschiebbar und medial ablenkbar zu sein; und
c) einen langgestreckten Stift (41), der medial bezüglich des Arms (42) angeordnet ist, wobei der langgestreckte Stift (41) bezüglich des Arms (42) zwischen einer arretierten Stellung, die eine mediale Ablenkung des Arms (42) verhindert, und einer nicht arretierten Stellung, in der eine mediale Ablenkung des Arms (42) gestattet ist, axial verschiebbar ist,
wobei das chirurgische Greiforgan (30) ausgelegt ist, um durch mediale Ablenkung des Arms (42) in vivo selektiv an dem zweiten Passmerkmal (43) des Arms (42) befestigt zu werden und in vivo davon entfernt zu werden.

2. Chirurgische Vorrichtung nach Anspruch 1, wobei
das zweite Passmerkmal (43) eine seitliche Einkerbung (43) an dem distalen Ende des langgestreckten Schafts (22) ist.

3. Chirurgische Vorrichtung nach Anspruch 2, wobei
das zweite Merkmal (33) auf dem chirurgischen Greiforgan (30) einen Ring (33) umfasst, der solche Abmessungen aufweist, dass er mit der seitlichen Einkerbung (43) zusammenpasst.

4. Chirurgische Vorrichtung nach Anspruch 1, umfassend zwei oder mehr Arme (42), die den langgestreckten Stift (41) umkreisen.

5. Chirurgische Vorrichtung nach Anspruch 1, wobei
das distale Ende des langgestreckten Stifts (41) eine Obturatorspitze umfasst.

6. Chirurgische Vorrichtung nach Anspruch 1, ferner umfassend einen Griff (21) in Wirkverbindung mit dem proximalen Ende des langgestreckten Schafts (22), wobei der Griff (21) einen Auslöser (54), der die axiale Bewegung des Arms (42) steuert, und ein Betätigungselement (53) umfasst, das die axiale Bewegung des langgestreckten Stifts (41) steuert.

7. Chirurgische Vorrichtung nach Anspruch 6, wobei
das Betätigungselement (53) arretierbar ist.

8. Chirurgische Vorrichtung nach Anspruch 1, wobei
das Greiforgan (30) zusammenarbeitende Backen aufweist, die sich als Reaktion auf eine axiale Bewegung des Arms (42) zwischen offenen und geschlossenen Stellungen bewegen.

9. Chirurgische Vorrichtung nach Anspruch 1, wobei der Arm (42) distal von dem distalen Ende des langgestreckten Schafts (22) vorragt.

10. Laparoskopische chirurgische Vorrichtung nach Anspruch 1, umfassend:
eine Vielzahl von Armen (42), die distal von dem distalen Ende des langgestreckten Schafts (22) vorragen, wobei die Arme (42) jeweils eine seitliche Einkerbung (43) als Passmerkmal umfassen, wobei die Arme (42) bezüglich des langgestreckten Schafts (22) axial verschiebbar und medial umlenkbar sind;
wobei der langgestreckte Stift (41) medial bezüglich der Arme (42) angeordnet ist, wobei der langgestreckte Stift (41) ausgelegt ist, um bezüglich der Arme (42) zwischen einer arretierten Stellung, die eine mediale Ablenkung des Arms (42) verhindert, und einer nicht arretierten Stellung, in der eine mediale Ablenkung des Arms (42) gestattet ist, axial verschiebbar zu sein; und
wobei das chirurgische Greiforgan (30) ausgelegt ist, um in vivo selektiv an dem Passmerkmal (43) der Arme (42) befestigt zu werden und in vivo davon entfernt zu werden, wobei das chirurgische Greiforgan (30) Backen aufweist, die sich als Reaktion auf eine axiale Bewegung des Arms (42) öffnen und schließen, wenn er an dem chirurgischen Greiforgan (30) befestigt ist.

11. Laparoskopische chirurgische Vorrichtung nach Anspruch 10, ferner umfassend eine Obturatorspitze an dem distalen Ende des langgestreckten Stifts (41).

## Revendications

1. Dispositif chirurgical comprenant :
un instrument (20) et un organe terminal effecteur chirurgical (30) pouvant, de manière sélective, être attaché à l'instrument (20) et être détaché de celui-ci, l'instrument (20) comprenant :
a) une tige allongée (22) définissant un axe longitudinal, la tige (22) comprenant une extrémité distale, une extrémité proximale et un premier élément d'accouplement (45) conçu pour s'accoupler avec un premier élément (32) sur l'organe terminal effecteur chirurgical (30) ;
b) un bras (42) comprenant un second élément d'accouplement (43) conçu pour s'accoupler avec un second élément (33) sur l'organe terminal effecteur chirurgical, le bras (42) étant conçu pour pouvoir coulisser axialement par rapport à la tige allongée (22) et pour pouvoir fléchir au niveau d'une section médiane ; et
c) une goupille allongée (41) se trouvant à une position médiane vis-à-vis du bras (42), la goupille allongée (41) pouvant coulisser axialement par rapport au bras (42) entre une position verrouillée empêchant un fléchissement au niveau de la section médiane du bras (42) et une position déverrouillée permettant un fléchissement au niveau de la section médiane du bras (42),
dans lequel l'organe terminal effecteur chirurgical (30) est conçu pour pouvoir, de manière sélective, être attaché *in vivo* au second élément d'accouplement (43) du bras (42) et être détaché *in vivo* de celui-ci, par le biais du fléchissement au niveau de la section médiane du bras (42).

2. Dispositif chirurgical selon la revendication 1, dans lequel le second élément d'accouplement (43) est une encoche latérale (43) au niveau de l'extrémité distale de la tige allongée (22).

3. Dispositif chirurgical selon la revendication 2, dans lequel le second élément (33) sur l'organe terminal effecteur chirurgical (30) comprend un anneau (33) dimensionné pour s'accoupler avec l'encoche latérale (43).

4. Dispositif chirurgical selon la revendication 1, comprenant au moins deux bras (42) entourant la goupille allongée (41).

5. Dispositif chirurgical selon la revendication 1, dans lequel l'extrémité distale de la goupille allongée (41) comprend une partie terminale formant obturateur.

6. Dispositif chirurgical selon la revendication 1, comprenant en outre une poignée (21) raccordée de manière fonctionnelle à l'extrémité proximale de la tige allongée (22), la poignée (21) comprenant une queue de détente (54) commandant le mouvement axial du bras (42) et un dispositif d'actionnement (53) commandant le mouvement axial de la goupille allongée (41).

7. Dispositif chirurgical selon la revendication 6, dans lequel le dispositif d'actionnement (53) peut être verrouillé.

8. Dispositif chirurgical selon la revendication 1, dans lequel l'organe terminal effecteur (30) comporte des mâchoires coopérantes qui se déplacent entre des positions ouverte et fermée en réaction à un mouvement axial du bras (42).

9. Dispositif chirurgical selon la revendication 1, dans lequel le bras (42) fait saillie dans le sens distal à partir de l'extrémité distale de la tige allongée (22).

10. Dispositif chirurgical laparoscopique selon la revendication 1, comprenant :
une pluralité de bras (42) faisant saillie dans le sens distal à partir de l'extrémité distale de la tige allongée (22), les bras (42) comprenant chacun une encoche latérale (43) en tant qu'élément d'accouplement, les bras (42) pouvant coulisser axialement par rapport à la tige allongée (22) et pouvant fléchir au niveau d'une section médiane ;
dans lequel la goupille allongée (41) se trouve à une position médiane vis-à-vis des bras (42), la goupille allongée (41) étant conçue pour pouvoir coulisser axialement par rapport aux bras (42) entre une position verrouillée empêchant un fléchissement au niveau de la section médiane des bras (42) et une position déverrouillée permettant un fléchissement au niveau de la section médiane des bras (42) ; et
dans lequel l'organe terminal effecteur chirurgical (30) est conçu pour pouvoir, de manière sélective, être attaché *in vivo* à l'élément d'accouplement (43) des bras (42) et être détaché *in vivo* de celui-ci, l'organe terminal effecteur chirurgical (30) comportant des mâchoires qui s'ouvrent et se ferment en réaction au mouvement axial des bras (42) lorsqu'il est attaché à l'organe terminal effecteur chirurgical (30).

11. Dispositif chirurgical laparoscopique selon la revendication 10, comprenant en outre une extrémité terminale formant obturateur sur l'extrémité distale de la goupille allongée (41).
